# EUROPEAN PATENT APPLICATION

(11) **EP 1 224 944 A1**
(43) Date of publication of application: **24.07.2002**
(21) Application number: 00964714.0
(22) Date of filing: 06.10.2000
(51) Int. Cl.: A61K 45/00, A61K 31/22, A61K 31/045, A61K 31/09, A61K 35/78, A61K 7/46, A61P 37/02, A61P 17/00

(54) **SKIN IMMUNE FUNCTION CONTROLLING AGENTS**

(30) Priority: 08.10.1999 JP 28789499
(71) Applicant: SHISEIDO COMPANY LIMITED, Tokyo 104-8010 (JP)
(72) Inventor: HOSOI, Junichi, Shiseido Research Center, Yokohama-shi, Kanagawa 236-8643 (JP); TSUCHIYA, Toru, Shiseido Research Center, Yokohama-shi, Kanagawa 236-8643 (JP)
(74) Representative: Albrecht, Thomas, Dr.
(86) International application number: JP0006980
(87) International publication number: WO0126686

(57) **Abstract**

Disclosed are skin immune function controlling agents which contain as the active ingredient a volatile substance capable of stimulating the olfactory receptor, as typified by a specific perfume. By controlling the skin immune function, these agents serve, for example, to enhance the skin barrier function and to prevent or treat skin contact hypersensitivity.

## Description

### Technical Field

This invention relates to the technical fields of cosmetics and dermatology. More particularly, this invention relates to preparations and methods for protecting the skin from external influences.

### Background Art

It has been known that aromatic plants and essential oils prepared therefrom exert various psychological and physiological influences on the mind and body of human beings. Volatile molecules of fragrance are received by a sensory protein present on the cilia of olfactory mucous cells, and signals are transferred to the limbic system and the hypothalamus through the medium of the olfactory bulb and the olfactory nervous system (Leonard and Tuite, Int. Rev. Neurobiol. 22:251-286, 1981).

Clinically, the psychotropic effects of perfumes have been tested (Komori et al., Neuroimmunomodulation 2:174-180). Thus, it has been clarified that an olfactory stimulus controls the systemic immune system as evidenced by a decrease of plaque-forming cells in anti-sheep erythrocytes (Shibata et al., Intern. J. Neuroscience 51:245-247, 1990) and an increase of sIgA in saliva (Komori et al., ibid.).

While the action of perfumes on the systemic immune system is being clarified as described above, the action of perfumes on the skin has been investigated solely by an analysis of electric potential changes on the skin surface (Miltner et al., Psychophysiology 31:107-110, 1994; Alaoui-Iamaili et al., Physiol. Behavior 62(4):713-720, 1997). Skin immunity is a defense function for preventing a foreign invasion and, at the same time, is a cause for allergy reactions. The skin immunocompetent cells called Langerhans' cells are dendritic cells located in the lower part of the epidermis, and induce or provoke immunity by presenting T cells with the antigen they have encountered.

Now, the effects of some specific perfumes are surveyed. It has been reported that citralva (i.e., 3,7-dimethyl-2,6-octadienenitrile) activates adenylate cyclase in the olfactory cells of frogs and rats (see Sklar et al., J. Biol. Chem. 261:15538-15543, 1986; and Boekhoff et al., EMBO J. 9:2453-2458, 1990, respectively). It has also been reported that a mixture of lyral [4-(4-hydroxy-4-methylpentyl)-3-cyclohexane-1-carboxyaldehyde] and lilial [4-(1,1-dimethylethyl)-α-methylbenzenepropanoll induces a metabolic turnover of inositol phosphate in olfactory receptor-expressing cells (Raming et al., Nature 361:353-356, 1993).

### Disclosure of the Invention

The present inventors have now found that certain volatile substances, which do not exhibit any substantial effect in the case of topical percutaneous administration, can control the skin immune reaction when they are placed in a state capable of stimulating the olfactory receptor or absorbed into the body through the respiratory tract. The present invention is based on this finding.

It has also been found that, on the other hand, the volatile substances capable of stimulating the olfactory receptor can be classified into substances which cause the sleeping hours of subjects to vary significantly in a barbiturate-induced sleep measuring method, and/or substances which cause the amplitude area of contingent negative variation (hereinafter referred to as CNV) of subjects to vary significantly in a CNV measuring method.

Accordingly, the present invention provides a skin immune function controlling agent which contains as the active ingredient a volatile substance capable of stimulating the olfactory receptor, more specifically, a substance that causes the sleeping hours of subjects to vary significantly in a barbiturate-induced sleep measuring method and/or a substance that causes the amplitude area of CNV of subjects to vary significantly in a CNV measuring method.

In another embodiment, the present invention provides the use of the volatile substance as an active ingredient for the preparation of a skin immune function controlling agent.

In still another embodiment, the present invention provides a method for controlling the skin immune function of a subject in which it is desired or required to control the skin immune function, the method comprising administering the volatile substance through the respiratory tract in an amount sufficient to control the immune function.

Since the present invention can control the skin immune function arbitrarily as desired, it is especially useful in preventing or treating an abnormality in the skin immune function (e.g., contact hypersensitivity) or in maintaining or recovering the skin barrier function.

### Brief Description of the Drawings

FIG. 1 is a graph showing the suppression of contact hypersensitivity when citralva was used as the active ingredient. "A" shows the results obtained by exposure to the perfume at the time of provocation of a contact hypersensitivity reaction, and "B" shows the results obtained by exposure to the perfume at the time of induction of a contact hypersensitivity reaction.
FIG. 2 is a graph showing a comparison between the skin immune suppressing effects of citralva and lyral/lilial.
FIG. 3 is a graph showing the skin immune suppressing effects obtained by the local administration of perfumes.
FIG. 4 is a graph showing the effect of the perfume DMMB in preventing a reduction in skin immunological competence due to restraint stress.
FIG. 5 is a graph showing the influence of stress imposition on Langerhans' cells, and the effect of the perfume DMMB in mitigating or suppressing the influence of stress imposition.

### Best Mode for Carrying Out the Invention

The "volatile substances capable of stimulating the olfactory receptor" which can be used in the present invention may be either natural substance or chemically synthesized substances, so long as they exhibit the action and effects meeting the purposes of the present invention. Moreover, they may be either substances having a fragrance (in particular, perfumes) or substance having no fragrance. In the present invention, the expression "capable of stimulating the olfactory receptor" means, for example, the action of binding to the receptors of olfactory cells and transferring signals to the center through the medium of a second messenger such as cyclic AMP. The term "volatile substances" refers to substances which are expelled as vapors at the living temperature of human beings or in a slightly warmed environment, and which can be kept in a liquid or solid state.

Such volatile substances include volatile substances which cause a significant variation in sleeping hours as measured by a barbiturate-induced sleep measuring method (see, for example, T. Tsuchiya et al., Brain Research Bulletin 26:379-401, 1991) that can generally be employed for the classification of perfumes and for other purposes, and volatile, substances which cause a significant variation in the amplitude area of contingent negative variation (hereinafter referred to as CNV) as determined by a CNV measuring method (see, for example, W.G. Walter et al., Nature 203:380-384, 1964; and S. Torii, Cosmetics and Toiletries 102, 1987).

It can be regarded as a measure of the aforesaid significant variation in sleeping hours that the sleeping hours measured for subjects treated with or exposed to a volatile substance is extended or shortened by at least 15%, as compared with the sleeping hours measured for subjects neither treated with nor exposed to the volatile substance (i.e., controls).

The volatile substances which serve to extend the sleeping hours include, but are not limited to, terpinyl acetate, phenetyl alcohol, linalool and anise (essential oil obtained from anise seeds). Generally, these volatile substances can stimulate a reduced skin immune function or prevent an abnormal reduction in skin immune function. On the contrary, the volatile substances which serve to shorten the sleeping hours include, but are not limited to, citralva, lemon oil (essential oil obtained from lemon peel), jasmine (absolute obtained from jasmine flowers), isobornyl acetate and phytol. Generally, these volatile substances can suppress an excessive or hypersensitive skin immune response:

On the other hand, it can be regarded as a measure of the aforesaid significant variation in the amplitude area of CNV measured for subjects treated with or exposed to a volatile substance is decreased or increased by at least 10%, as compared with the amplitude area of CNV measured for subjects neither treated with nor exposed to the volatile substance (i.e., controls).

The volatile substances which decrease the amplitude area include, but are not limited to, caraway, 1,3-dimethoxy-5-methylbenzene (DMMB), sandalwood (essential oil obtained from sandalwood), rose (essential oil or absolute obtained from rose flowers), rosemary (essential oil obtained from the whole plant of rosemary), bergamot (essential oil obtained from bergamot peel), lavender (essential oil obtained from the spikes of lavender), orange (essential oil obtained from orange peel) and chamomile (essential oil obtained from dried chamomile flowers).

Generally, these volatile substances can stimulate the aforesaid skin immune function. On the contrary, the volatile substances which increase the amplitude area include, but are not limited to, lemon oil (essential oil obtained from lemon peel), jasmine (absolute obtained from jasmine flowers), peppermint (essential oil obtained from the whole plant of peppermint), basil (essential oil obtained from the whole plant of basil), clove (essential oil obtained from the flower buds of clove) and star anise (essential oil obtained from star anise fruit). Generally, these volatile substances can suppress the aforesaid skin immune function (i.e., can suppress an excessive skin immune response). The subjects mentioned in connection with the aforesaid measuring methods can usually be healthy adult humans or mammals other than humans (e.g., mice and rats).

In the skin immune function controlling agents of the present invention, one of the aforesaid volatile substances or a mixture of two or more of the aforesaid volatile substances may be used as the active ingredient. As described above, the term "skin immune function control" as used herein means suppressing immunity when skin immunity is excessively stimulated and, on the other hand, stimulating or activating immunity when skin immunity is weakened. Moreover, the term "skin immune function control" also comprehends preventing the immune function regarded as normal from being excessively stimulated or weakened.

In order to secure the aforesaid effects of the present invention, it is necessary to place the volatile substance constituting the active ingredient in a state capable of stimulating the olfactory receptor or to cause an effective amount thereof to be absorbed into the body through the respiratory tract. Such an effective amount can be defined, for example, as an amount capable of decreasing or increasing the number of Langerhans' cells significantly as compared with controls (untreated subjects), when evaluated according to the testing method which will be described later. In the aforesaid volatile substances which shorten the sleeping hours and/or increase the amplitude area of CNV, the effective amount is an amount capable of decreasing the number of Langerhans' cells significantly. In the other type of volatile substances, the effective amount is an amount capable of producing the opposite effect.

Such an effective amount cannot be clearly specified, because the optimum amount may vary according to the age and immune state of the subject, the dosage, administration time and administration method of the perfume, and the like. Generally, a specialist can determine the effective amount by reference to the testing methods and results which will be described later. Moreover, it is desirable that the effective amount should be determined by a specialist. Furthermore, when the aforesaid volatile substances which shorten the sleeping hours and/or increase the amplitude area of CNV are used to prevent or treat a contact hypersensitivity reaction, the effective amount thereof can also be determined by reference to the methods for evaluating a contact hypersensitivity reaction in model animals as will be described later and the results obtained thereby.

The agents of the present invention may be made into preparations (or products) by mixing them with carriers, diluents and other active ingredients which are commonly used in perfume preparations. The dosage form may be any of various forms commonly used for perfumes, provided that the volatile substance can be absorbed or administered into the body through the respiratory tract. Examples thereof include fragrance products such as perfumes, Cologne water and room aromatics; and cosmetic preparations such as lotions, creams, soaps, dentifrices and aerosols. Furthermore, they may have the form of specific pharmaceutical preparations such as inhalations. For example, when a specific volatile substance is incorporated into a pharmaceutical preparation for preventing or treating skin contact hypersensitivity, the amount of volatile substance used may be such that its inhalation is sufficiently effective in suppressing contact hypersensitivity. The volatile substance is generally incorporated in an amount of not less than 0.01% by weight, though it depends on the dosage form and the type of drug (perfume).

The dosage form and other conditions have been described above, with principal consideration for application to human beings. However, the preparations and controlling methods of the present invention may be applied to mammals other than human beings, such as cattle, pigs, sheep, rabbits and rats. When they are applied to these mammals, the above-described dosage form and other conditions may be modified to some extent.

The present invention is more specifically explained with reference to the following examples. These examples are intended to specify indices to the effects exhibited by the present invention. However, it is to be understood that the present invention is not limited thereto.

### Test Example 1

Influence of perfumes on a contact hypersensitivity reaction and the like

### (Experimental animals)

Female C57 BL/6 mice (aged 7-9 weeks) were used. Each group of five animals were placed in a cage (225 × 335 × 140 mm) and kept in a chamber maintained at a temperature of 21-25°C and a relative humidity of 40-70%. The inhalation of a perfume into the animals was carried out by placing a Petri dish having a diameter of 10 cm in the cage, putting therein a filter paper impregnated with the perfume, and covering the dish with wire netting. (A filter paper impregnated with no perfume was used for a control group.) This animal experiment followed the National Research Council Guidelines.

### 1-1. Contact hypersensitivity reaction

On day 1, mice were sensitized by clipping the hair in a dorsal region of each mouse and applying thereto 2,4,6-trinitrochlorobenzene (TNCB) (50 µl of a 3% acetone solution) as an antigen. After 5 days (on experimental day 6), a contact hypersensitivity reaction was provoked by applying 5 µl of a 1% acetone solution of the aforesaid antigen to both sides of the right ear of each animal. The contact hypersensitivity (hereinafter referred to as CH) reaction was evaluated by measuring the thickness of the swollen left and right ears on day 7 with a micrometer and calculating the difference between the measured values.

In order to examine the effect of perfume inhalation at the time of sensitization, a Petri dish containing a filter paper impregnated with the aforesaid perfume was placed in the cage over a period of time extending from day 0 to day 2. In order to examine the effect of perfume inhalation at the time of immunity provocation, the aforesaid Petri dish was placed in the cage over a period of time extending from day 5 to day 7. In order to examine the effect of the direct application of a perfume to the skin, 50 µl of 0.1% citralva, lyral/lilial or diluent (50% corn oil in acetone) was applied to a region to be sensitized, and the aforesaid antigen was applied thereto after 6 hours. The long-term effect was tested by applying 50 µl of 1% perfume or diluent every day over a period of time extending from day 0 to day 2.

### Results:

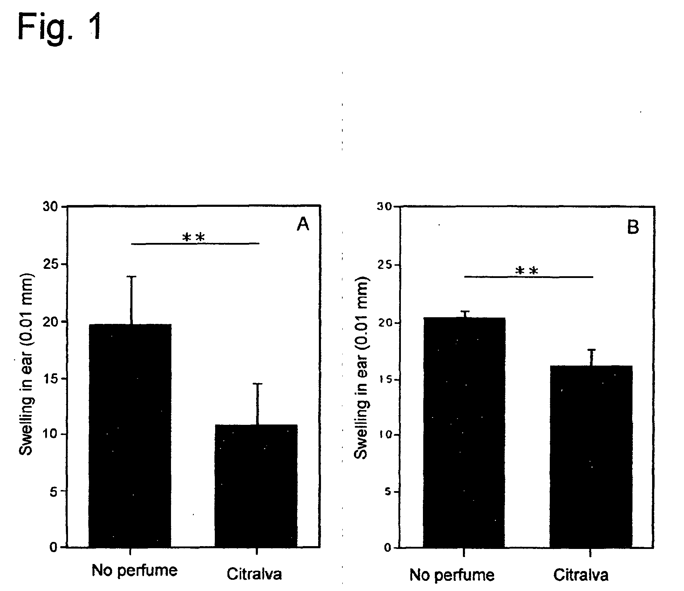

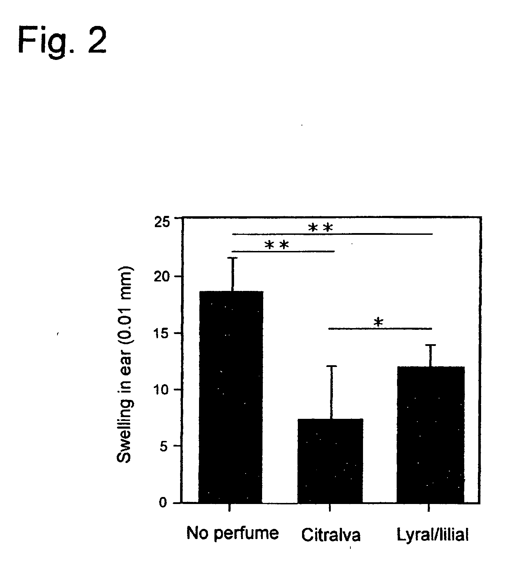

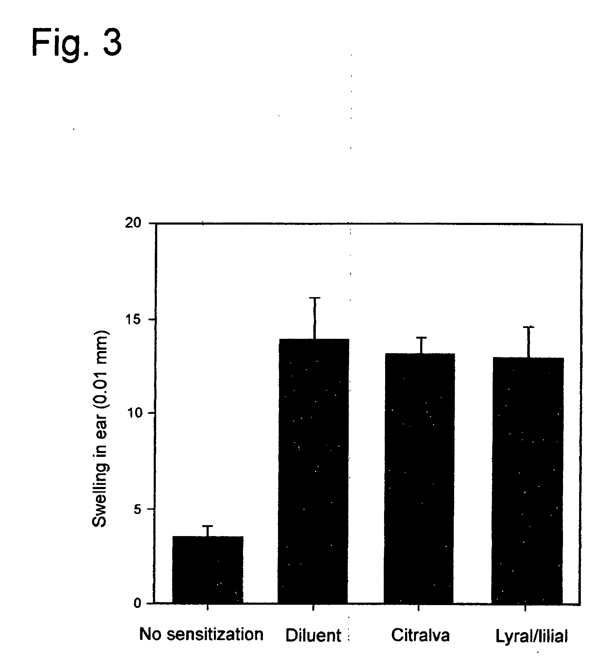

(1) The results obtained by exposing mice to citralva for 2 days at the time of inducing or provoking a CH reaction with TNCB are shown in FIG. 1. It can be seen from FIG. 1 that the inhalation of citralva suppresses the provocation of the CH reaction by about 50% as compared with the controls (A). It, can also be seen that the inhalation of citralva significantly suppresses the induction of the CH reaction by about 20% (B). These results suggest that an olfactory stimulus can control a skin immune reaction.
(2) The results obtained by examining the effects of perfumes on the provocation of a CH reaction are shown in FIG. 2. This figure shows a comparison of the effects of citralva (a perfume which shortens the aforesaid sleeping hours) and lyral/lilial falling under another category. It can be seen that both perfumes suppress the CH reaction, but the effect of citralva is significantly more powerful than that of lyral/lilial. It can also be seen that the suppression of the CH reaction is influenced by the type of perfume.
   The above-described experimental results were obtained by subjecting the measured results for each group of five mice to a statistical analysis using the t-test.
(3) The effects of the topical application of perfumes are shown in FIG. 3. It can be seen from this figure that neither citralva nor lyral/lilial exerts an influence on the CH reaction.

### 1-2. Staining of Langerhans' cells

Epidermal Langerhans' cells were stained according to the well-known method. In brief, an ear of a mouse was incubated in a 3.8% aqueous solution of ammonium thioisocyanate at 37°C for 20 minutes to separate the epidermis from the corium. The epidermal sheet was fixed with acetone and incubated in the culture supernatant of mouse hybridoma M5/114.15.2 (American Tissue Type Collection, Rockville, MD, USA) so as to cause the secretion of anti-I-A IgG. Then, the anti-I-A IgG was combined with fluorescein isothiocyanate (FITC) (Sigma, USA)-labeled anti-mouse IgG antibody. Cells positive for the I-A antigen were observed under a confocal laser scanning microscope (MRC600; Bio Rad, USA) and analyzed. Samples were collected from each group of five mice. Three regions of each sample were analyzed by a blind test.

### Results:

After exposure to citralva, samples were collected, stained for the I-A antigen, and observed under a confocal laser scanning microscope. The results of analysis are shown in Table I [in this table, the values in parentheses are standard deviations (SDs)].

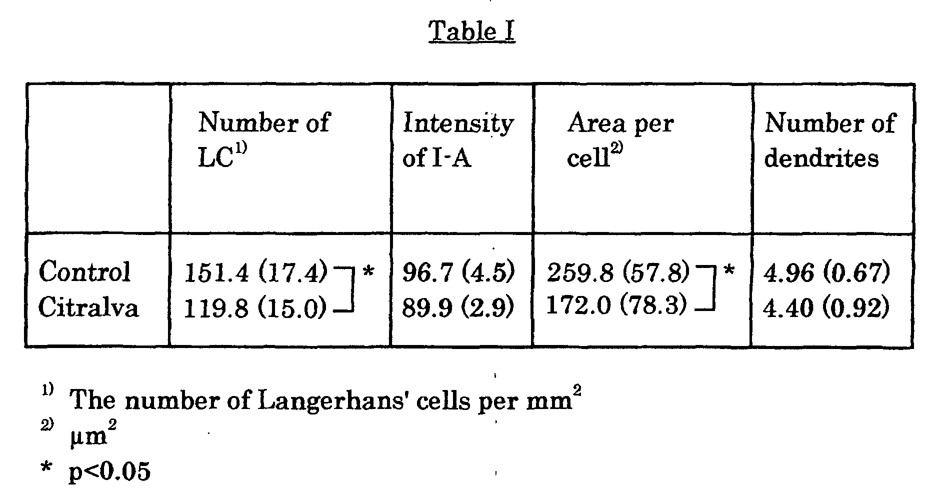

After animals were kept in the presence of citralva or lyral/lilial, epidermal sheets were collected, stained for I-A, and observed under a confocal laser scanning microscope. The results of analysis are shown in Table II [in this table, the values in parentheses are standard deviations (Sds)].

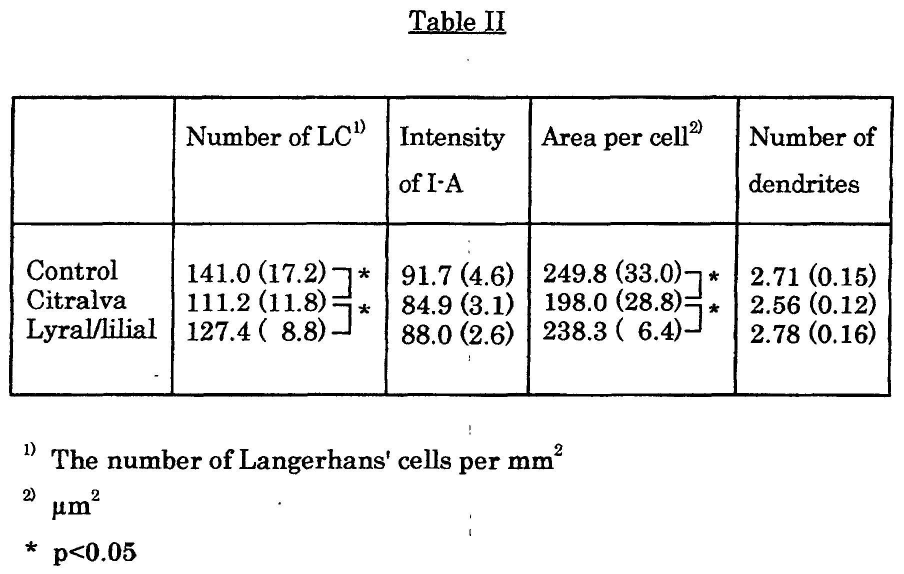

It can be seen from the above tables that the number of LC is significantly decreased after exposure to citralva (Table I).

It can also be seen that the area of each I-A-positive cell is significantly reduced by exposure to citralva. A comparison of the effects of perfumes has revealed that citralva exhibits a more powerful effect than lyral/lilial (Table II).

These results mean that an olfactory stimulus exerts an influence on epidermal Langerhans' cells.

### Test Example 2

Effect of a perfume on the suppression of a skin immune reaction by stress imposition

### (Experimental animals)

Female C57 BL/6 mice (aged 7-9 weeks) (purchased from Charles River Japan) were used. Each group of five animals were placed in a cage (225 × 335 × 140 mm) and kept in a chamber maintained at a temperature of 21-25°C and a relative humidity of 40-70%. The inhalation of a perfume into the animals was carried out by placing a Petri dish having a diameter of 10 cm in the cage, putting therein a filter paper impregnated with the perfume, and covering the dish with wire netting. (A filter paper impregnated with no perfume was used for a control group.) These animals were laid in a prone position and wrapped in a pliable fine-meshed wire net for 6 hours. This animal experiment gained the approval of the animal experimentation committee of our company following the National Research Council Guidelines.

### 2-1. Contact hypersensitivity reaction

Mice were sensitized by applying 2,4,6-trinitrochlorobenzene (TNCB) (10 µl of a 3% acetone solution) to the inner and outer surfaces of the left ear of each mouse. After 5 days, a CH reaction was provoked by applying TNCB (5 µl of a 1% acetone solution) to the inner and outer surfaces of the right ear of each mouse. After 24 hours, the swelling was evaluated by measuring the thicknesses of both ears with a micrometer (manufactured by Mitsutoyo) and subtracting the thickness of the left ear from that of the right ear. The exposure of the animals to a perfume was continued over a period of time extending from 24 hours before provocation to the measurement of the swelling. The restraint stress was imposed 24 hours before provocation and repeated immediately after provocation. This experiment was carried out by using the animals in groups of five and using 1,3-dimethoxy-5-methylbenzene (DMMB; purchased from Takasago International Inc.) as the perfume.

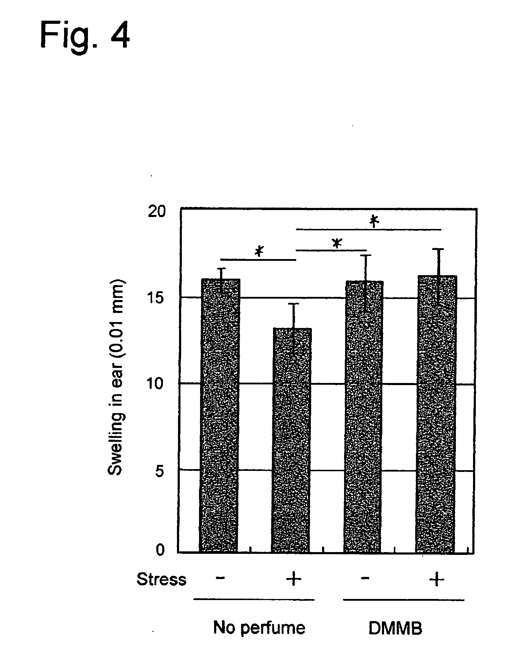

The results thus obtained are shown in Table III below and FIG. 4.

**Table III**

| (1) Measured values | | | |
|---|---|---|---|
| Swelling* (× 0.01 mm) | | | |
| Unrestrained, no perfume | Restrained, no perfume | Unrestrained, DMMB | Restrained, DMMB |
| 16 | 15 | 16 | 15 |
| 15 | 14 | 17 | 14 |
| 16 | 13 | 13 | 17 |
| 17 | 11 | 17 | 17 |
| 16 | 13 | 16 | 18 |

| (2) Analysis of variance (one-way layout) | | | | |
|---|---|---|---|---|
| Group | Number of samples | Sum | Average | Variance |
| Unrestrained, no perfume | 5 | 80 | 16 | 0.5 |
| Restrained, no perfume | 5 | 66 | 13.2 | 2.2 |
| Unrestrained, DMMB | 5 | 79 | 15.38 | 2.7 |
| Restrained, DMMB | 5 | 81 | 16.2 | 2.7 |

The values given in Table III, (1) are values obtained by subtracting the measured thickness of the left ear of each test animal from that of the right ear thereof. The results obtained by subjecting these values to an analysis of variance are shown in Table III, (2).

As can be seen from the above Table III and FIG. 4, a skin immune reaction was suppressed by stress imposition, but the suppression of the skin immune reaction by stress was not observed in the animal group having undergone DMMB inhalation. This means that a certain perfume can prevent the skin immune function from being reduced by stress.

### 2-2.Staining of Langerhans' cells

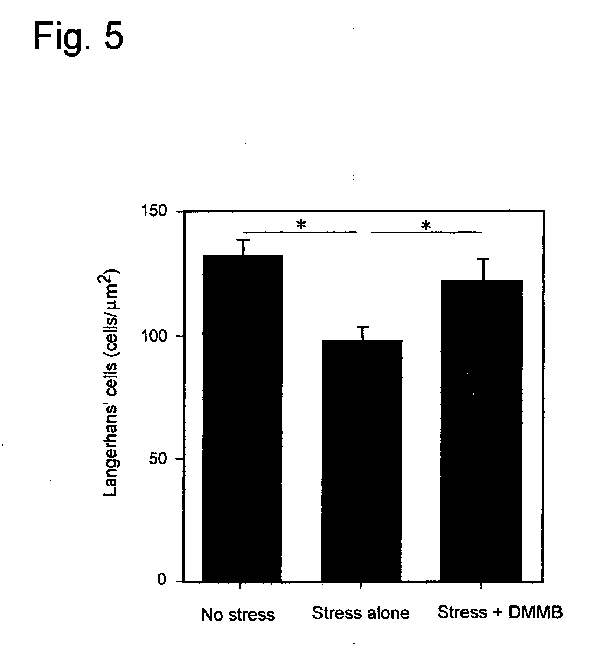

Epidermal Langerhans' cells were stained and observed in the same manner as described in Example 1, "1-2. Staining of Langerhans' cells". The results of analysis are shown in FIG. 5.

It can be seen from this figure that, as a result of stress imposition, the number of Langerhans' cells in the epidermis was decreased by about 30%. It can also be seen that, when the test animals were made to absorb DMMB at the time of stress imposition, the number of Langerhans' cells was significantly increased as compared with the animal group having undergone stress imposition alone, and was of the same order as that of the animal group having undergone no stress imposition.

### Industrial Applicability

The present invention provides preparations and methods for controlling the skin immune function by using certain volatile substances. Since these preparations and methods may be realized, for example, in a wide variety of cosmetic preparations and cosmetological techniques, they can be utilized in the industries for the manufacture of cosmetics and toiletry products and in the beauty art.

## Claims

1. A skin immune function controlling agent which contains as the active ingredient a volatile substance capable of stimulating the olfactory receptor.

2. An agent as claimed in claim 1 wherein the volatile substance causes the sleeping hours of subjects to vary significantly in a barbiturate-induced sleep measuring method.

3. An agent as claimed in claim 2 wherein the volatile substance extends the sleeping hours of subjects by at least 15% as compared with controls.

4. An agent as claimed in claim 2 wherein the volatile substance extends the sleeping hours of subjects by at least 15% as compared with controls, and is selected from the group consisting of terpinyl acetate, phenetyl alcohol, linalool and anise.

5. An agent as claimed in claim 2 wherein the volatile substance shortens the sleeping hours of subjects by at least 15% as compared with controls.

6. An agent as claimed in claim 2 wherein the volatile substance shortens the sleeping hours of subjects by at least 15% as compared with controls, and is selected from the group consisting of citralva, lemon oil, jasmine oil, isobornyl acetate and phytol.

7. An agent as claimed in claim 1 wherein the volatile substance causes the amplitude area of contingent negative variation (CNV) of subjects to vary significantly in a CNV measuring method.

8. An agent as claimed in claim 7 wherein the volatile substance decreases the amplitude area of CNV of subjects by at least 10% as compared with controls.

9. An agent as claimed in claim 7 wherein the volatile substance decreases the amplitude area of CNV of subjects by at least 10% as compared with controls, and is selected from the group consisting of caraway, 1,3-dimethoxy-5-methylbenzene (DMMB), sandalwood, rose, rosemary, bergamot, lavender, orange and chamomile.

10. An agent as claimed in claim 7 wherein the volatile substance increases the amplitude area of CNV of subjects by at least 10% as compared with controls.

11. An agent as claimed in claim 7 wherein the volatile substance increases the amplitude area of CNV of subjects by at least 10% as compared with controls, and is selected from the group consisting of lemon oil, jasmine, peppermint, basil, clove and star anise.

12. The use, as an active ingredient for the preparation of a skin immune function controlling agent, of a volatile substance which causes the sleeping hours of subjects to vary significantly in a barbiturate-induced sleep measuring method, or a volatile substance which causes the amplitude area of CNV of subjects to vary significantly in a CNV measuring method.

13. A method for stimulating the skin immune function of a subject in which it is desired or required to stimulate the skin immune function, the method comprising administering a volatile substance through the respiratory tract in an amount sufficient to stimulate the immune function, the volatile substance comprising a substance which extends the sleeping hours of subjects significantly in a barbiturate-induced sleep measuring method and/or a substance which decreases the amplitude area of CNV of subjects significantly in a CNV measuring method.

14. A method for suppressing the skin immune function of a subject in which it is desired or required to suppress the skin immune function, the method comprising administering a volatile substance through the respiratory tract in an amount sufficient to suppress the immune function, the volatile substance comprising a substance which shortens the sleeping hours of subjects significantly in a barbiturate-induced sleep measuring method and/or a substance which increases the amplitude area of CNV of subjects significantly in a CNV measuring method.

15. A method as claimed in claim 13 or 14 which is applied to human skin care for cosmetological purposes.
